(19) Europäisches Patentamt
European Patent Office
Office européen des brevets

(11) Publication number: **0 112 637**
**A2**

# (12) EUROPEAN PATENT APPLICATION

(21) Application number: 83306963.6

(22) Date of filing: 15.11.83

(51) Int. Cl.³: **C 07 D 405/14**

(30) Priority: 25.11.82 GB 8233676
25.11.82 GB 8233675

(43) Date of publication of application: 04.07.84
Bulletin 84/27

(84) Designated Contracting States: AT BE CH DE FR GB IT LI LU NL SE

(71) Applicant: SMITH KLINE & FRENCH LABORATORIES LIMITED, Mundells, Welwyn Garden City Hertfordshire, AL7 1EY (GB)

(72) Inventor: Brown, Thomas Henry, 17 Godfries Close, Tewin Hertfordshire (GB)
Inventor: Durant, Graham John, Pengelly 15A Briary Wood Lane, Welwyn Hertfordshire (GB)
Inventor: Walsgrove, Timothy Charles, 80 Pondcroft Road, Knebworth Hertfordshire (GB)
Inventor: White, George Raymond, 13 Fairfield Close, Harpenden Hertfordshire (GB)

(74) Representative: Waters, David Martin, Dr. et al, Smith Kline & French Laboratories Ltd. Patent Department Mundells, Welwyn Garden City Hertfordshire AL7 1EY (GB)

(54) **Chemical process for preparing H2-antagonists.**

(57) A chemical process is described for the preparation of $H_2$-antagonists or precursors thereof, of the formula (V):

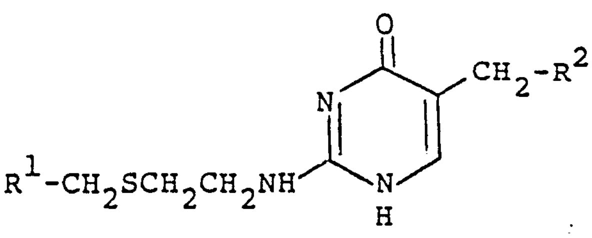

(V)

wherein $R^1$ is 5-methylimidazol-4-yl, furan-2-yl or 5-dimethylaminomethylfuran-2-yl and $R^2$ is 3,4-methylenedioxyphenyl or optionally protected 2-hydroxypyrid-4-yl; which process comprises reacting a compound of the formula (VI) with a compound of the formula (VII):

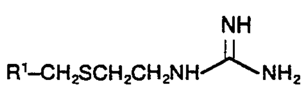

(VI)

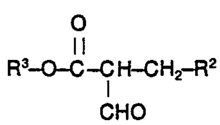

(VII)

wherein $R^3$ is $C_{1-6}$alkyl; and thereafter if desired deprotecting a protected hydroxy group, converting a compound wherein $R^1$ is furan-2-yl into a compound wherein $R^1$ is 5-dimethylaminomethylfuran-2-yl, and/or forming a pharmaceutically acceptable salt.

EP 0 112 637 A2

# CHEMICAL PROCESS FOR PREPARING $H_2$-ANTAGONISTS

This invention relates to a chemical process, and in particular to a process for the preparation of a small class of histamine $H_2$-antagonists. A particular compound of interest that is prepared by this process is 2-[2-(5-methyl-4-imidazolylmethylthio)ethylamino]-5-(3,4-methylenedioxybenzyl)pyrimidin-4-one (known as oxmetidine) or an acid addition salt thereof. Another particular compound of interest is 2-[2-(5-dimethylaminomethylfuran-2-ylmethylthio)ethylamino]-5-(2-hydroxypyrid-4-ylmethyl)pyrimidin-4-one or an acid addition salt thereof.

In the UK Patent Specifications 1,595,291 and 1,582,527 and in EP-A-3677 and EP-A-4793 general processes are described for the preparation of histamine $H_2$-antagonists, which comprise the displacement by an amine of a leaving group from the 2-position of a pyrimidinone. For example oxmetidine is exemplified in UK Patent Specification 1,595,291 as being prepared by the reaction of a compound of the formula (I) with a compound of the formula (II) wherein Q is methylthio :

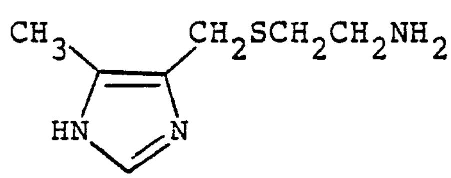

(I)

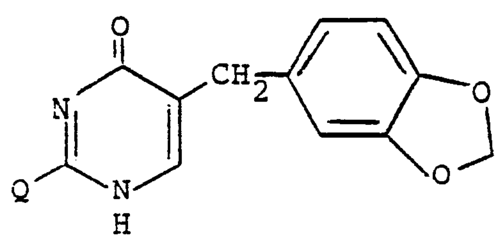

(II)

and EP-A-4793 exemplifies a process which comprises the reaction of a compound of the formula (I) with a compound of the formula (II) wherein Q is nitroamino ($NO_2NH$-).

The hereinbefore referred to furan derivative is exemplified in EP-A-3677 as being prepared by the reaction of a compound of the formula (III) with a compound of the formula (IV) followed by the conversion of the methoxy group to a hydroxy group :

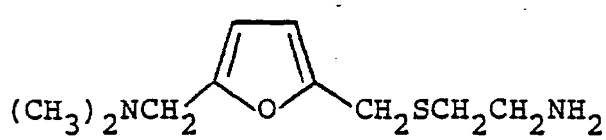

(III)

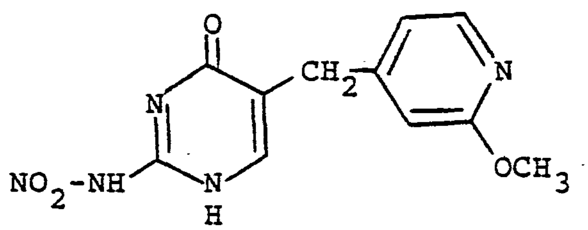

(IV)

The reaction of the compound of the formula (I) and the compound of the formula (II) wherein Q is methylthio presents an environmental disadvantage in that the unpleasant methyl mercaptan is produced as a by-product. The preparation of the compound of the formula (II) wherein Q is nitroamino, or the compound of the formula (IV), involves the use of nitroguanidine which has been reported to explode after detonation of the dry solid. Thus the preparations of the compounds containing a nitroamino group represent disadvantageous aspects of the reaction between the compound of the formula (I) and the compound of the formula (II) wherein Q is nitroamino, and the reaction of the compounds of the formulae (I) and (IV).

According to the present invention there is provided a process for the preparation of a compound of the formula (V) or an acid addition salt thereof :

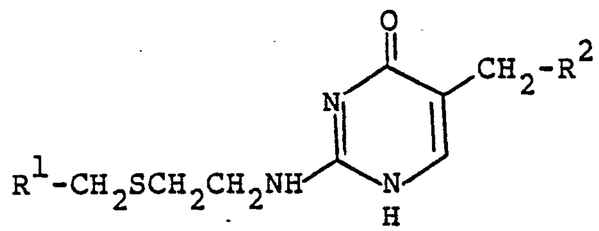

(V)

wherein $R^1$ is 5-methylimidazol-4-yl, furan-2-yl or 5-dimethylaminomethylfuran-2-yl and $R^2$ is 3,4-methylenedioxyphenyl or optionally protected 2-hydroxypyrid-4-yl;

which process comprises reacting a compound of the formula (VI) or acid addition salt thereof :

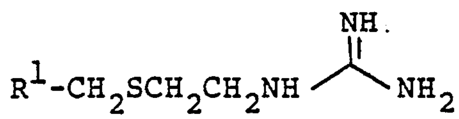

(VI)

wherein $R^1$ is as defined hereinbefore, with a compound of the formula (VII) :

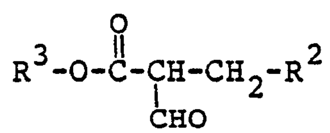

(VII)

wherein $R^2$ is as defined hereinbefore and $R^3$ is $C_{1-6}$alkyl; and thereafter if desired :

i) deprotecting a protected hydroxy group;

ii) converting a compound wherein $R^1$ is furan-2-yl into a compound wherein $R^1$ is 5-dimethylaminomethylfuran-2-yl,

iii) forming a pharmaceutically acceptable salt.

Suitably $R^1$ is 5-methylimidazol-4-yl. Suitably $R^1$ is furan-2-yl or 5-dimethylaminomethylfuran-2-yl.

Suitably $R^2$ is 3,4-methylenedioxyphenyl. Suitably $R^2$ is optionally protected 2-hydroxypyrid-4-yl. Suitable protected hydroxy groups include $C_{1-6}$alkoxy, benzyloxy and substituted benzyloxy groups. Such groups may be converted to hydroxy in conventional manner for example by acidic hydrolysis and hydrogenolysis.

An especially preferred value for $R^2$ is 2-methoxypyrid-4-yl.

In a preferred aspect of this invention $R^1$ is 5-methylimidazol-4-yl and $R^2$ is 3,4-methylenedioxyphenyl.

In another preferred aspect of this invention $R^1$ is furan-2-yl or 5-dimethylaminomethylfuran-2-yl and $R^2$ is optionally protected 2-hydroxypyrid-4-yl. Favourably $R^2$ is 2-methoxypyrid-4-yl.

The reaction is conveniently performed in a solvent in which the reactants are substantially inert and are soluble enough to react, for example a $C_{1-6}$alkanol, an ether solvent such as tetrahydrofuran or dimethoxyethane, dimethylformamide, dimethylacetamide, dimethylsulphoxide, ethyl acetate or mixtures of such solvents, and optionally in the presence of water.

Preferably the reaction is performed in a $C_{1-6}$alkanol for example methanol, ethanol, 1-propanol, 2-propanol or 1-butanol, of these methanol is favoured.

Preferably in the compounds of the formula (VII) $R^1$ is methyl or ethyl, favourably ethyl.

Suitably substantially equimolar quantities of the compound of the formula (VI) and the compound of the

formula (VII) are reacted. The compound of the formula (VI) is conveniently generated in situ from an acid addition salt for example a sulphate.

The reaction is suitably performed under basic conditions. If an acid addition salt of the compound of the formula (VI) is used, then about a mole equivalent of base is required to generate the free base of the compound of the formula (VI). Suitably the reaction of the free base of the compound of the formula (VI) with the compound of the formula (VII) is carried out in the presence of a base, which may be less than one equivalent, one equivalent or more than one equivalent; for example between 0.25 and 5 equivalents. We have found two equivalents to be convenient, so that if an acid addition salt of the compound of formula (VI) is used, about three equivalents of base are generally used in the overall process.

Suitably the base used in this process is an alkali metal hydroxide, carbonate or $C_{1-6}$alkoxide. Preferably the base is a sodium or potassium $C_{1-6}$alkoxide, for example sodium methoxide, sodium ethoxide, sodium n-propoxide, sodium isopropoxide, potassium methoxide, potassium ethoxide, potassium n-propoxide or potassium isopropoxide. Of these sodium methoxide is favoured.

The reaction may be performed at a temperature of from ambient to reflux. Suitably reflux temperatures, for example in methanol, for between 4 and 24 hours are convenient.

The skilled man will appreciate that the compounds of the formulae (VI) and (VII) may react to give isomeric 1- and 3-substituted-2-aminopyrimidinones in addition to the desired compound. Surprisingly it has been found

that the desired product is obtained in good yield with only a small proportion of the undesired isomeric by-products. The desired product can be purified in conventional manner.

The process of this invention avoids the disadvantages of the exemplified prior art processes referred to hereinbefore.

One particularly advantageous manner of purification comprises the evaporation of the crude reaction mixture to afford an oily and/or solid residue. Water is then mixed with this residue, the pH of the resultant aqueous layer being approximately 12, and the aqueous layer decanted off. The decanted aqueous layer contains the desired product and by-products to approximately the same extent as does the residue, as adjudged by standard techniques, for example chromatography. Thus no purification is effected in the above procedure. If, however, the pH of the aqueous layer is lowered, to a pH below 8 for example about 5 to 8, more suitably about 6 to 8, after mixing and decanting (or extraction into organic solvent), the decanted aqueous layer contains very little of the desired product and substantially all of the impurities, the residue being surprisingly free of impurities. The fact that there is no purification when the aqueous layer is at a pH of about 12 and that a very effective purification is achieved when the pH is about 5 to 8 is unexpected and of significant advantage. Thus this is a preferred feature of the process of the present invention.

Suitably the pH of the aqueous layer is lowered using an aqueous acid, for example hydrochloric acid or acetic acid.

The compound of the formula (V) may be converted to a pharmaceutically acceptable acid addition salt in conventional manner for example using an equivalent of the desired acid in an organic solvent, for example isopropanol or ethanol.

The conversion of a compound of the formula (V) wherein $R^1$ is furan-2-yl to a compound of the formula (V) wherein $R^1$ is 5-dimethylaminomethylfuran-2-yl may be performed by the methods disclosed in EP-A-3677. The conversion of a compound of the formula (V) wherein $R^2$ is a protected 2-hydroxypyrid-4-yl group to a compound of the formula (V) wherein $R^2$ is a 2-hydroxypyrid-4-yl group may be performed in conventional manner, selected as appropriate by the skilled man, for example by the methods of EP-A-3677. It should be realised that when $R^1$ is furan-2-yl certain methods of conversion of a protected hydroxy group to a hydroxy group may be inappropriate, for example treatment with concentrated mineral acid, as the unsubstituted furan-2-yl group is liable to decomposition at low pH values.

The compounds of the formula (VI) and acid addition salts thereof are prepared in conventional manner, for example by reaction of a compound of the formula (VIII) with a compound of the formula (IX) :-

$R^1-CH_2SCH_2CH_2NH_2$

(VIII)

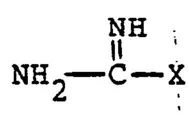

(IX)

wherein $R^1$ is as hereinbefore defined and X is a leaving group, for example $C_{1-6}$alkylthio, preferably methylthio. Alternatively the compounds of the formula (VI) may be prepared by the reaction of a compound of formula (VIII)

with cyanamide (for example 50% aqueous cyanamide) conveniently at reflux and conveniently at a slightly basic pH.

The compounds of the formula (VII)* are preparable by the methods of EP-A-3677 and EP-A-4793.

The following Description and Examples serve to illustrate this invention.

Description 1

A mixture of 2-[(2-furylmethyl)thio]ethylamine hemisulphate (1600 g) and n-propanol (2.8 L) was heated to 50°C, to which mixture was added 50% aqueous cyanamide (400 ml). The pH was adjusted to 7.9 with 10N sodium hydroxide solution. The reaction mixture was heated to reflux. Additional 400 ml portions of aqueous cyanamide were added at 1 hour, 2 hours and 5 hours maintaining the pH at 8.3 ± .2 using 10 N sodium hydroxide and 6N sulphuric acid. After 7.5 hours at reflux, the reaction was diluted with isopropanol (3.8 L) and then filtered whilst hot. The filtrate was diluted again with isopropanol (6 L) (40-43°) and stirred for 16 hours as it cooled. Cooling to 5-8° afforded 2-[(2-furylmethyl)-thio]ethylguanidine hemisulphate (1528 g), yield 79%.

Description 2

N-[2-(5-Methyl-4-imidazolylmethylthio)ethyl]guanidine sulphate

2-(5-Methyl-4-imidazolylmethylthio)ethylamine (300g) and S-methyl-thiouronium sulphate (225.7 g) were refluxed in water (2400 ml) for 3½ hours. The solution was cooled, evaporated under reduced pressure to 500 ml and

acidified to pH 2 with 2N sulphuric acid. Ethanol was added to bring a solid out of solution, this solid was flitered, dried and recrystallised from aqueous methanol to yield the title product (419 g), m.p. 174-176°C.

## EXAMPLES

### Example 1

A methanolic solution of sodium methoxide (6.75 ml) (prepared from sodium (2.299 g) in methanol (50 ml)) was added to ethyl 2-formyl-3-(3,4-methylenedioxy)phenylpropionate (1.125 g) in methanol (10 ml) with stirring. To the resulting stirred yellow solution was added N-[2-(5-methyl-4-imidazolylmethylthio)ethyl]guanidine sulphate (1.400 g) in portions over 30 minutes. The resulting mixture was refluxed overnight, cooled to ambient temperature and evaporated under reduced pressure to afford a pale yellow glass. Water (20 ml) was added whereupon an oil came out of solution. 2N Hydrochloric acid was added dropwise, resulting in a white precipitate, until the pH was 8.0. The mixture was left to stand overnight, treated with a further small amount of hydrochloric acid and filtered to afford an oily solid. This solid was mixed with 1-propanol (20 ml) containing water (4 ml), and ethanolic HCl (10 ml) was added. This mixture was warmed to boiling point, filtered through charcoal and diatomaceous earth, the filtrate was collected, reduced in volume by boiling and subsequently cooled to 20°C. Trituration afforded a solid which was washed with 1-propanol (2 ml) and diethyl ether to afford 2-[2-(5-methyl-4-imidazolylmethylthio)ethylamino]-5-(3,4-methylenedioxybenzyl)-4-pyrimidinone dihydrochloride (1.20 g, 57%); recrystallisation from aqueous 1-propanol/ethanolic HCl afforded material with melting point 228-228.5°C.

Example 2

To a solution of sodium methoxide (146.8 g) in methanol (1200 ml) was added ethyl 2-formyl-3-(3,4-methylenedioxy)phenylpropionate (225 g) in methanol (1200 ml). N-[2-(5-Methyl-4-imidazolylmethylthio)ethyl]-guanidine sulphate (296 g) was added and the resultant mixture was refluxed for 20 hours. The reaction mixture was cooled and filtered with the solids being thoroughly washed and stirred with further methanol (800 ml). The filtrate was evaporated under reduced pressure to yield an oil. To this oil was added water (2000 ml) and the pH was adjusted to 7.5 with concentrated hydrochloric acid. After standing overnight the aqueous layer was decanted and the oil dissolved with refluxing in a mixture of 1-propanol (2000 ml) and concentrated hydrochloric acid (220 ml). Approximately one litre of solvent was removed by distillation and the remainder of the mixture was cooled to 15°C whereupon oily crystals formed. 1-Propanol (600 ml) was added to the mixture with warming to dissolve the crystals, and on recooling crystals were formed which were filtered off and washed with 1-propanol (300 ml) to afford 2-[2-(5-methyl-4-imidazolylmethylthio)ethylamino]-5-(3,4-methylenedioxybenzyl)-4-pyrimidinone dihydrochloride (151.4 g, 36%); recrystallisation from aqueous 1-propanol afforded material with a melting point of 233-234.5°C. Crystallisation of the evaporated mother liquors from aqueous methanol afforded a further amount of product (27.7 g).

Example 3

To a stirred mixture of ethyl 2-formyl-3-(3,4-methylenedioxy)phenylpropionate (33.75 g) and N-[2-(5-ethyl-4-imidazolylmethylthio)ethyl]guanidine sulphate (44.2 g) in

methanol (330 ml) was added solid sodium methoxide (22.94 g). The mixture was refluxed for 22 hours, cooled and evaporated under reduced pressure to yield an oil. This oil was taken up in water (250 ml) and the pH of this solution adjusted to 7.5 by the addition of concentrated hydrochloric acid. On standing an oil separated out, the aqueous layer was decanted and the oil 'washed' with warm water. The oil was dissolved with warming in 1-propanol (300 ml) and filtered through charcoal whilst warm. To the resultant solution was added concentrated hydrochoric acid (24 ml), a proportion of the solvent (200 ml) was removed by distillation and on cooling the concentrated solution a solid crystallised out and was filtered and washed with 1-propanol to afford 2-[2-(5-methyl-4-imidazolylmethylthio)ethylamino]-5-(3,4-methylenedioxybenzyl)-4-pyrimidinone dihydrochloride (20 g, 55%).

Example 4

To a stirred solution of sodium methoxide (97 g) in methanol (800 ml) was added ethyl 2-formyl-3-(3,4-methylenedioxy)phenylpropionate (148 g) in methanol (800 ml). To the resultant solution was added N-[2-(5-methyl-4-imidazolylmethylthio)ethyl]guanidine sulphate (193.7 g). The mixture was refluxed for 20 hours and evaporated under reduced pressure to give a semi-crystalline brown oil. This oil was mixed with water (1600 ml) and the pH adjusted to 7.5 with concentrated hydrochloric acid. The aqueous layer was decanted off and the oil was dissolved in a mixture of 1-propanol (1300 ml) and concentrated hydrochloric acid (118 ml). This solution was refluxed, filtered through charcoal, cooled and the solvent was evaporated under reduced pressure to yield yellow oily crystals which were slurried with methanol (200 ml) and collected to afford 2-[2-(5-methyl-4-imidazolylmethylthio)ethylamino]-5-(3,4-methylenedioxybenzyl)-4-pyrimidinone dihydrochloride (127.6 g).

Example 5

A solution of ethyl 2-formyl-3-(3,4-methylenedioxy)-phenylpropionate (236.4 g) in methanol (500 ml) was added to sodium methoxide (138 g) in methanol (1130 ml). To this solution was added N-[2-(5-methyl-4-imidazolylmethylthio)ethyl]guanidine sulphate (277.3 g) in methanol (2300 ml) and the mixture was refluxed for 20 hours. The mixture was evaporated under reduced pressure to yield a semi-crystalline brown oil. This oil was stirred with water (2000 ml), warmed to about 40°C, and the pH was adjusted to 7.5 with concentrated hydrochloric acid. The mixture was cooled, the aqueous layer decanted and the remaining oil was dissolved in 1-propanol (1200 ml)/concentrated hydrochloric acid (168 ml). The solvents were evaporated under reduced pressure and crystallisation performed from 1-propanol to yield 2-[2-(5-methyl-4-imidazolylmethylthio)ethylamino]-5-(3,4-methylenedioxybenzyl)-4-pyrimidinone dihydrochloride (223.8 g, 56%), melting point 227-230°C.

Example 6

2-[[(Furan-2-ylmethyl)thio]ethylamino]-5-[2-methoxypyridin-4-yl)methyl]pyrimidin-4-one

i) Ethyl-2-formyl-3(2-methoxypyridin-4-yl)propionate

Ethyl 3-(2-methoxypyridin-4-yl)propionate (2.6 g) and ethyl formate (1.48 g) in anhydrous dimethylformamide (10 ml) were added to a suspension of sodium hydride (0.8 g; 50% oil suspension) in anhydrous dimethylformamide (10 ml) at ice-bath temperature. The stirred mixture was allowed to warm slowly to room temperature and left for 16 hours. The solution was diluted with water (100 ml),

washed with dichloromethane (3 x 20 ml), taken to pH 5.5 with acetic acid and extracted into dichloromethane (4 x 20 ml). The combined organic extracts were dried over $MgSO_4$, filtered and evaporated under reduced pressure to afford ethyl 2-formyl-3-(2-methoxypyridin-4-yl)-propionate as an oil.

ii) <u>2-[[(Furan-2-ylmethyl)thio]ethylamino]-5-[(2-methoxypyridin-4-yl)methyl]pyrimidin-4-one</u>

The ethyl 2-formyl-3-(2-methoxypyridin-4-yl) propionate from part i) above was stirred with 2-[(furan-2-ylmethyl)thio]ethylguanidine hemisulphate (3.23 g) in propan-1-ol (25 ml) at 50°C under a nitrogen atmosphere. Sodium methoxide (two equivalents) was added and stirring was continued at 50°C for 60 minutes, followed by heating at 90°C for 20 hours. The mixture was allowed to cool, water (2 ml) was added and subsequently acetic acid (a few drops) was added to take the pH to 5.5-6.0. The mixture was evaporated under reduced pressure, dissolved in water (20 ml) and the product extracted, at approximately pH 5.5, into dichloromethane (4 x 20 ml). The combined organic extracts were washed with water at pH 5.5, dried over $MgSO_4$, filtered and evaporated under reduced pressure to give a red gum which gradually solidified. This was crystallised from propan-2-ol/water to afford 2-[[(furan-2-ylmethyl)thio]ethylamino]-5-[(2-methoxypyridin-4-yl)-methyl]pyrimidin-4-one (1.0 g), melting point 117-117.5°C.

<u>Claims</u> :-

1. A process for the preparation of a compound of the formula (V) or an acid addition salt thereof :

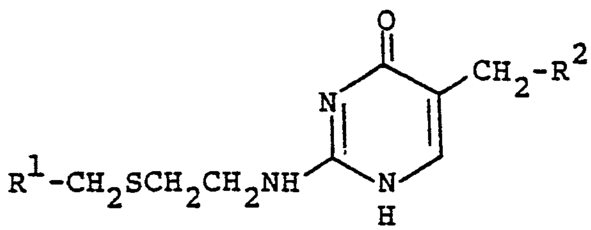

(V)

wherein $R^1$ is 5-methylimidazol-4-yl, furan-2-yl or 5-dimethylaminomethylfuran-2-yl and $R^2$ is 3,4-methylenedioxyphenyl or optionally protected 2-hydroxypyrid-4-yl;

which process comprises reacting a compound of the formula (VI) or acid addition salt thereof :

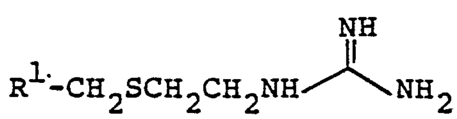

(VI)

wherein $R^1$ is as defined hereinbefore, with a compound of the formula (VII) :

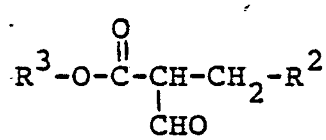

(VII)

wherein $R^2$ is as defined hereinbefore and $R^3$ is $C_{1-6}$alkyl; and thereafter if desired :

i) deprotecting a protected hydroxy group,

ii) converting a compound wherein $R^1$ is furan-2-yl into a compound wherein $R^1$ is 5-dimethylaminomethylfuran-2-yl,

iii) forming a pharmaceutically acceptable salt.

2. A process according to claim 1 wherein $R^3$ is ethyl.

3. A process according to either claim 1 or claim 2 when performed in methanol.

4. A process according to claim 3 when performed at reflux.

5. A process according to any one of claims 1 to 4 wherein the reaction is performed in the presence of sodium methoxide.

6. A process according to any one of claims 1 to 5 wherein the compound of the formula (VI) is in the form of a sulphate.

7. A process according to any one of claims 1 to 6 wherein $R^1$ is furan-2-yl or 5-dimethylaminomethylfuran-2-yl and $R^2$ is optionally protected 2-hydroxypyrid-4-yl.

8. A process according to any one of claims 1 to 6 wherein $R^1$ is 5-methylimidazol-4-yl and $R^2$ is 3,4-methylenedioxyphenyl.

9. A process according to any one of claims 1 to 7 wherein $R^2$ is pyrid-4-yl substituted in the 2-position by $C_{1-6}$alkoxy, benzyloxy or substituted benzyloxy.

10. A process according to claim 9 wherein $R^1$ is furan-2-yl and $R^2$ is 2-methoxypyrid-4-yl.

11. A process according to any one of claims 1 to 10 wherein purification comprises the mixing of a crude reaction mixture with water at a pH of about 5 to 8 and subsequently removing the aqueous layer.

12. 2-[[(Furan-2-ylmethyl)thio]ethylamino]-5-[(2-methoxypyridin-4-yl)methyl]pyrimidin-4-one.